# EUROPÄISCHE PATENTSCHRIFT

(11) **EP 0 655 916 B1**
(45) Veröffentlichungstag und Bekanntmachung des Hinweises auf die Patenterteilung: **04.02.1998**
(21) Anmeldenummer: 93919108.6
(22) Anmeldetag: 19.08.1993
(51) Int. Cl.: A61K 31/565, A61K 9/70

(54) **MITTEL ZUR TRANSDERMALEN APPLIKATION ENTHALTEND 3-KETO-DESOGESTREL**
TRANSDERMAL APPLICATION AGENT CONTAINING 3-KETO-DESOGESTREL
AGENT D'APPLICATION TRANSDERMIQUE CONTENANT DU 3-CETO-DESOGESTREL

(30) Priorität: 21.08.1992 DE 4227989
(43) Veröffentlichungstag der Anmeldung: 07.06.1995
(73) Patentinhaber: SCHERING AKTIENGESELLSCHAFT, 13342 Berlin (DE)
(72) Erfinder: LIPP, Ralph, D-10717 Berlin (DE); GÜNTHER, Clemens, D-13357 Berlin (DE); RIEDL, Jutta, D-14057 Berlin (DE); TÄUBER, Ulrich, D-13359 Berlin (DE)
(86) Internationale Anmeldenummer: EP9302224
(87) Internationale Veröffentlichungsnummer: WO9404157

(56) Entgegenhaltungen:
- EP-A- 0 137 278
- EP-A- 0 253 607
- EP-A- 0 275 716
- EP-A- 0 491 438
- EP-A- 0 491 443
- CONTRACEPTION Bd. 42, Nr. 1 , 1990 Seiten 1 - 11 S.-E. OLSSON ET AL. 'Clinical results with subcutaneous implants containing 3-keto desogestrel'
- M. ROSOFF 'Controlled release of Drugs: Polymers and Aggregate Systems' 1989 , VCH PUBLISHERS , USA siehe Seite 277 - Seite 305
- LANCET Bd. 335, Nr. 8685 , 1990 Seiten 310 - 312 M.I. WHITEHEAD ET AL. 'Transdermal administration of oestrogen/progestagen hormone replacement therapy'

## Beschreibung

Die Erfindung betrifft ein Mittel zur transdermalen Applikation, dadurch gekennzeichnet, daß es 3-Keto-desogestrel gegebenenfalls in Kombination mit einem oder mehreren Östrogen(en) enthält.

3-Keto-desogestrel (13-Ethyl-11-methylen-17β-hydroxy-18,19-dinor-17α-pregnen-3-on) ist eine Substanz der Formel

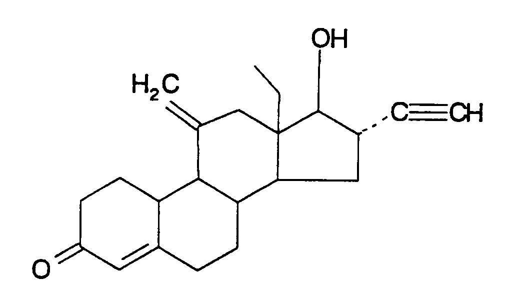

Es ist bekanntlich eine pharmakologisch wirksame Verbindung mit außergewöhnlich starker gestagener Wirksamkeit, welche in Form seines Prodrugs Desogestrel (J. of Steroid Biochem., 14, 1981, 175 ff und Europ. J. Clin. Pharmakol 15, 1979, 349 ff) in Kombination mit östrogen wirksamen Verbindungen zur Herstellung von oral zu applizierenden Mitteln konzeptionsverhindernder Wirkung (Marvelon ® ) verwendet wird.

Es wurde nun gefunden, daß 3-Keto-desogestrel gegebenenfalls in Kombination mit einem oder mehreren Östrogen(en) sehr gut zur Herstellung eines Mittels für die transdermale Applikation des Wirkstoffes oder Wirkstoffgemisches verwendet werden kann. Grundsätzlich könnte man auch das Desogestrel selbst transdermal applizieren. 3-Keto-desogestrel ist aber wegen seiner höheren Wirkpotenz vorzuziehen.

Transdermal zu applizierende Arzneimittel haben bekanntlich den Vorzug, daß sie über einen längeren Zeitraum hin eine gleichmäßigere Freisetzung des Wirkstoffs ermöglichen, als dies in der Regel bei anders - wie zum Beispiel peroral - zu applizierenden Mitteln möglich ist. Diese Eigenschaften lassen sich in einer Reihe von endokrinen Erkrankungen vorteilhaft ausnutzen. Für in Wasser schwer lösliche Steroidhormone, wie zum Beispiel die Gestagene ist es aber in der Regel recht problematisch, transdermale Systeme zu erstellen, die eine zur Therapie ausreichende Penetration des Wirkstoffs durch die Haut gewährleisten.

Es wurde nun gefunden, daß es mit Hilfe des erfindungsgemäßen Mittels überraschenderweise möglich ist, eine therapeutisch ausreichende sehr gleichmäßige Penetrationsgeschwindigkeit der Steroidhormone durch die Haut zu erzielen, wahrend dies bei den bekannten Steroidhormone enthaltenden transdermal zu applizierenden Mitteln nur bedingt möglich ist. (EP-A 137278 und EP-A 275716), was den Einsatz von vergleichsweise großen Systemen notwendig macht.

Geeignete Östrogene für das erfindungsgemäße Mittel sind beispielsweise das Estradiol, das Estriol, das Ethinylestradiol, das Mestranol, das 14α,17α-Ethanoestra-1,3.5(10)-trien 3β,17α-diol (WO 88/01275), das 14α,17α-Ethanoestra-1,3,5(10)-trien-3β,16α,17α-triol (WO91/08219)
und deren Ester (EP-A 163596), wie das Estradiol-dipropionat, das Estradiol-dihexanoat und das Estradiol-decanoat. Die erfindungsgemäßen Kombinationspräparate enthalten neben 3-Keto-desogestrel vorzugsweise 1 bis 3 - insbesondere 1 bis 2 Östrogen(e).

Zur Herstellung pharmazeutischer Präparate kann der Wirkstoff oder das Wirkstoffgemisch in geeigneten flüchtigen Lösungsmitteln und/oder penetrationsverstärkenden Mitteln gelöst oder suspendiert werden. Die erhaltenen Lösungen oder Suspensionen können mit den üblichen Hilfsstoffen, wie Matrixbildnern und Bakteriziden versetzt und gegebenenfalls nach Sterilisation in übliche Dosierbehältnisse abgefüllt werden. Andererseits ist es aber auch möglich, diese Lösungen oder Suspensionen unter Einbeziehung von Emulgatoren und Wasser zu Lotionen oder Salben weiterzuverarbeiten. Man kann auch - gegebenenfalls unter Zugabe von Treibgas - Sprays herstellen, die in den üblichen Dosierbehältnissen abgefüllt werden können.

Geeignete flüchtige Lösungsmittel sind beispielsweise niedere Alkohole, Ketone oder niedere Carbonsäureester wie Ethanol, Isopropanol, Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Geeignete penetrationsverstärkende Mittel sind beispielsweise ein- oder mehrwertige Alkohole, wie Ethanol, 1,2-Propandiol oder Benzylalkohol, gesättigte und ungesättigte Fettalkohole mit 8 bis 18 Kohlerstoffatomen, wie Laurylalkohol oder Cetylalkohol, Kohlenwasserstoffe, wie Mineralöl, gesättigte und ungesättigte Fettsäuren mit 8 bis 18 Kohlenstoffatomen, wie Stearinsäure oder Ölsäure, Fettsäureester mit bis zu 24 Kohlenstoffatomen oder Dicarbonsäurediester mit bis zu 24 Kohlenstoffatomen.

Fettsäureester, die sich für das erfindungsgemäße Mittel eignen, sind beispielsweise solche der Essigsäure, Capronsäure, Laurinsäure, Myristinsäure, Stearinsäure und Palmitinsäure, wie zum Beispiel die Methylester, Ethylester, Propylester, Isopropylester, Butylester, sec.-Butylester, Isobutylester, tert.-Butylester oder Monoglycerinsäureester dieser Säuren. Besonders bevorzugte Ester sind solche der Myristinsäure, oder Ölsäure wie deren Methylester und insbesondere deren Isopropylester. Geeignete Dicarbonsäurediester sind beispielsweise das Diisopropyladipat, Diisobutyladipat und Diisopropylsebacat.

Weitere penetrationsverstärkende Mittel sind Phosphatidderivate, wie das Lecitin, Terpene, Amide, Ketone, Harnstoff und seine Derivate oder Ether wie zum Beispiel Dimethylisosorbid und Diethylenglycolmonoethylether. Es bedarf keiner näheren Erläuterung, das auch Gemische dieser penetrationsverstärkenden Mittel zur Herstellung des erfindungsgemäßen Mittels geeignet sind.

Die Konzentration, in welcher der Wirkstoff- oder das Wirkstoffgemisch optimalerweise in dem Lösungsmittel gelöst oder suspendiert werden, beträgt für 3-Keto-desogestrel üblicherweise 0,01 bis 25 Gewichtsprozent. Bei den Östrogenen ist die Konzentration naturgemäß von der Art des verwendeten Wirkstoffs und der angestrebten Einzeldosis abhängig, sie muß im Einzelfall mittels der dem Fachmann geläufigen Vorversuche, wie zum Beispiel der Bestimmung der erreichbaren Plasmakonzentrationen an Wirkstoff nach dermaler Applikation, bei ausgewählten erfindungsgemäßen Mitteln ermittelt werden. Im allgemeinen werden auch hier Wirkstoffkonzentrationen von 0,01 bis 25 Gewichtsprozent Östrogen im erfindungsgemäßen Mittel ausreichend sein. Das Gewichtsverhältnis von 3-Keto-desogestrel zu dem oder den Östrogen(en) liegt bei den Kombinationspräparaten bei 5:1 bis 1:10.

Für die orale Kontrazeption beträgt die gestagene Tagesdosis 150µg Desorgestrel, welches während der Absorption nahezu vollständig in das pharmakologisch aktive 3-keto-Desogestrel umgewandelt wird. Die kontrazeptive Tagesdosis konnte durch Einarbeitung von 3-keto-Desogestrel in ein subkutanes Depot (Implanon ®) (Contraception 47, 1993, 251-261), aufgrund einer relativ konstanten Freigaberate auf ca. 60µg 3-keto-Desogestrel gesenkt werden. Um diese Tagesdosis von 60µg 3-keto-Desogestrel auf transdermalem Wege mit einem TTS von 10cm² Fläche bioverfügbar zu machen, wird ein transdermaler Fluß von ca. 250 ng/cm²/h benötigt.

Die Bestimmung des Ausmaßes der Geschwindigkeit der percutanen Resorption von [3H]-3-Keto-desogestrel durch die Vollhaut einer haarlosen Maus kann mittels der Franz-Durchflußkammer ermittelt werden.

Mit Hilfe der Franz-Kammer-Batterie wird der Zeitverlauf der [3H]-Radioaktivität in der Rezeptor-Flüssigkeit, blutisotonem Polyethylenglykol 400, die 1000 I.E. Penicillin pro ml enthält, bestimmt.

Pro Ansatz werden drei Experimente durchgeführt, in denen jeweils zwei Formulierungen in drei Franz-Kammern untersucht, die mit Vollhaut einer haarlosen Maus bespannt sind untersucht werden. Je Hautfeld (0,32 cm²) werden 200µg 3-Keto-desogestrel, die in 10µl Formulierung gelöst bzw. suspendiert sind, mit einer Eppendorfpipette appliziert.

Die Probeentnahme aus der Rezeptorflüssigkeit erfolgt alle zwei Stunden, insgesamt 48 Stunden lang.

Die nachfolgende Tabelle zeigt die in diesen Versuchen erhaltenen Ergebnisse.

| **Percutaner Fluß von 3-Keto-desogestrel-haltigen Formulierungen in ng/cm**^{**2**}**/h** | | | | |
|---|---|---|---|---|
| Versuch | Formulierung in | mittlerer Fluß am 1ten Tag | mittlerer Fluß am 2ten Tag | max. Fluß |
| A | 1,2-Propandiol | 920 ± 539 | 1316 ± 572 | 1975 ± 765 |
| B | 95% 1,2-Propandiol + 5% Laurocapsam* | 3606 ± 695 | 3748 ± 468 | 4440 ± 808 |
| C | 90% 1,2-Propandiol + 10% Laurinsäure | 3855 ± 994 | 4892 ± 928 | 5033 ± 942 |
| D | 90% 1,2-Propandiol + 10% Laurylalkohol | 4885 ± 1261 | 5245 ± 928 | 5899 ± 1361 |
| E | Isopropylmyristat | 2184 ± 354 | 2743 ± 193 | 3002 ± 247 |
| F | Propylenglykol-monolaurinsäureester | 1531 ± 562 | 2464 ± 530 | 2626 ± 599 |

| | | | | |
|---|---|---|---|---|
| * Azone® der Firma Nelson Corp. | | | | |

Man sieht, daß die therapeutisch erforderliche Tagesdosis muhelos erreicht oder sogar überschritten wird.

Eine sehr gleichmäßige Applikation mit eingestellter Dosierung des Wirkstoffes oder Wirkstoffgemischen kann man erzieien, wenn man den Wirkstoff oder das Gemisch in ein transdermales therapeutisches System (TTS) einbettet. Geeignete transdermale therapeutische Systeme sind solche, die man üblicherweise zur percutanen Applikation von Wirkstoffen anwendet (Yie W. Chien: "Transdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Research 1030 Hamilton Court Menlo Park CA 94025 (415) 328-2228).

So kann man beispielsweise ein solches transdermales therapeutisches System verwenden, welches aus
a) einer undurchlässigen Deckschicht,
   ein bis drei an der Deckschicht haftenden, das 3-Keto-desogestrel gegebenenfalls das oder die Östrogen(e) und gewünschtenfalls penetrationsverstärkende Mittel enthaltende, für diese Komponenten durchlässigen selbsthaftenden oder von einem gewünschtenfalls penetrationsverstärkende Mittel enthaltenden Hauthaftkleber abgedecktem oder umgebenen Matrixschicht(en), einer abziehbaren Schutzschicht, oder
b) einer mit einem gewünschtenfalls penetrationsverstärkende Mittel enthaltenden Haftkleber versehen Abdeckung,
   ein bis drei (jeweils) eine Haftkleberumrandung unbedeckt lassende, mittels einer un durchlässigen Abdeckung an dem Haftkleber befestigten, das 3-Keto-desogestrel, gegebenenfalls des oder die Östrogen(e) und penetrationsverstärkende Mittel enthaltende Matrixschicht(en) und einer abziehbaren Schutzschicht, oder
c) einer undurchlässigen Deckschicht,
   ein bis drei an oder in der Deckschicht befindlichen, das 3-Keto-desogestrel, gegebenenfalls das oder die Östrogen(e) und gewünschtenfalls penetrationsverstärkende Mittel enthaltenden Arzneimitelreservoir(e),
   ein bis drei für diese Komponenten durchlässigen Polymerschicht(en) einer durchlässigen gegebenenfalls penetrationsverstärkende Mittel enthaltende Hauthaftkleber-Schicht und einer abziehbaren Schutzschicht besteht.

Ein transdermales therapeutisches System gemäß Variante a) stellt ein einfaches Matrixsystem dar. Es kann beispielsweise von runder, ovaler oder rechteckiger Form sein und wie folgt hergestellt werden.

Eine Lösung oder Suspension von bis zu 25 Gewichtsprozent Wirkstoff oder Wirkstoffgemisch, 0-40 Gewichtsprozent eines penetrationsverstärkenden Mittels, 30-70 Gewichtsprozent eines medizinisch üblichen Klebers aufgefüllt mit einem geeigneten flüchtigen Lösungsmittels zu 100 Gewichtsprozent wird auf eine plane undurchlässige Deckschicht gestrichen. Nach dem Trocknen kann auf diese Schicht eine zweite und gewünschtenfalls später sogar eine dritte gegebenenfalls Wirkstoff, penetrationsverstärkende Mittel und Kleber enthaltende Schicht aufgetragen und getrocknet werden. Dann wird das Matrixsystem mit einer abziehbaren Schutzschicht versehen.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusätzlich mit einem Hauthaftkleber abdecken oder umgeben.

Geeignete Lösungsmittel und penetrationsverstärkende Mittel sind beispielsweise die bereits erwähnten Flüssigkeiten dieser Art. Als medizinisch übliche Kleber eigenen sich beispielsweise Polyacrylate, Silicone, Polyurethane, Blockpolymere, Styrol-Butadien-Kopolymere sowie natürliche oder synthetische Kautschuke, wie z.B. Polyisobutylene. Als weitere Matrixbildner kommen Celluloseether, Polyvinylverbindungen oder Silikate in Betracht. Zur Erhöhung der Klebrigkeit können der erhaltenen Matrix die üblichen Additive, wie zum Beispiel klebrig machende Harze und Öle zugesetzt werden.

Als Schutzschicht eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielswiese 10 bis 100 µm dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise über eine Flache von 5 bis 100 cm².

Bei mehrschichtigen Matrixsystemen kann beispielsweise in die an der undurchlässigen Deckschicht aufgetragenen Matrix das 3-Keto-desogestrel und gegebenenfalls die Penetrationsverstärker eingebracht werden, während die darunter befindliche Schicht oder Schichten die Estrogene und gegebenenfalls ebenfalls Penetrationsverstärker enthält. Andererseits ist es aber auch möglich in einem solchen transdermalen System mehrere wirkstoffhaltige Matrixsysteme nebeneinander anzuordnen.

Ein transdermales therapeutisches Matrixsystem gemäß Variante b kann beispielsweise ebenfalls rund, oval oder rechteckig sein und wie folgt hergestellt werden.

Eine Abdeckung wird mit einem Hauthaftkleber beschichtet. Dann klebt man auf diese pro TTS ein bis drei ausgestanzte Areale einer mit einer undurchlässigen Abdeckung versehenen, das 3-Keto-desogestrel, gegebenenfalls das oder die Östrogen(e) und penetrationsverstärkende Mittel enthaltende Matrixschichten so auf, daß die Abdeckung einen ausreichenden Rand zur Befestigung auf der Haut und bei mehreren Arealen auch ausreichende Zwischenräume besitzt und versieht sie mit einer abziehbaren Schutzschicht. Die in diesen Matrixsystem verwendeten Materialien können die gleichen sein, wie in denjenigen der Variante a.

Ein transdermales therapeutisches Reservoirsystem gemäß Variante c kann beispielsweise ebenfalls rund, oval oder rechteckig sein und wie folgt dargestellt werden;

Eine undurchlässige Folie wird durch Warme und/oder Zug so verformt, daß eine bis drei 0,1 bis 3 ml fassende Ausbuchtungen entstehen. Diese wird mit einer wirkstoffhaltigen Lösung oder Suspension enthaltend 1-50 Gewichtsprozent Wirkstoff oder Wirkstoffgemisch mit einem penetrationsverstärkenden Mittel gefüllt. Die wirkstoffhaltige Lösung oder Suspension kann auch mit bis zu 10 Gewichtsprozent Matrixbildner verdickt sein.

Als Abdeckung des Reservoirs zur Haut hin dient eine aufgeschweißte oder aufgeklebte durchlässige Polymerschicht, auf welche eine durchlässige Hauthaftkleberschicht und eine abziehbare Schutzschicht angebracht wird.

Es können bei diesem System die oben erwähnten penetrationsverstärkenden Mittel angewendet werden. Als durchlässige Polymerschicht wird beispielsweise eine 20 bis 200 µm dicke Folie aus Celluloseestern, Celluloseethern, Siliconen oder Polyolefinverbindungen verwendet. Durch Variation dieser Polymerschicht läßt sich die Diffusionsgeschwindigkeit des Wirkstoffes oder Wirkstoffgemisches innerhalb weiter Grenzen variieren.

Als Kleber und Schutzschicht eigenen sich die gleichen Materialien, die bei dem transdermalen therapeutischen System gemäß Variante a beschrieben sind.

Bei der Herstellung von transdermalen therapeutischen Systemen mit zwei oder drei nebeneinander angeordneten wirkstoffhaltigen Matrixschichten oder Arzneimittelreservoiren ist es oft zweckmäßig in dem einen das 3-Keto-desogestrel und in dem anderen das oder die Östrogene einzubringen. In derartigen Fallen können die wirkstoffhaltigen Matrixsysteme oder Arzneimittelreservoire nicht nur unterschiedliche Wirkstoffe sondern zusätzlich auch noch unterschiedliche penetrationsverstärkende Mittel enthalten.

Im Falle der Matrixsysteme gemäß Variante a oder b muß man für einen ausreichenden Abstand der Areale Sorge tragen um eine Diffusion der Wirkstoffe in das jeweils andere Areal zu unterbinden. Im Falle der Reservoirsysteme gemäß Variante c ist es möglich, die einzelnen Reservoire mit unterschiedlich durchlässiten Polymerschichten zu versehen um so den Diffusionsfluß der einzelnen Wirkstoffe den jeweiligen Bedürfnissen anzupassen.

Weitere Merkmale der erfindungsgemäßen transdermalen Systeme seien anhand der beigefügten, nicht maßstabgerechten Zeichnungen erläutert.

Fig. 1 zeigt einen Querschnitt durch ein einfaches rundes Matrixsystem gemäß Variante a ohne die abziehbare Schutzschicht. Es besteht aus der undurchlässigen Deckschicht 1 und der arzneimittelhaltigen Matrixschicht 2.

Fig. 2 zeigt einen Querschnitt durch ein Matrixsystem gemäß Variante b ohne die abziehbare Schutzschicht.

Fig. 3 zeigt einen Längsschnitt durch dieses System. Das System besteht aus der Abdeckung 3, die mit einer Haftkleberschicht 4 versehen ist. An dieser Haftkleberschicht sind mittels undurchlässiger Abdeckungen 5 und 7 zwei arzneimittelhaltige Matrixschichten 6 und 8 befestigt.

Fig. 4 zeigt einen Querschnitt durch ein rundes einkammeriges Reservoirsystem gemäß Variante c ohne die abziehbare Schutzschicht. Es besteht aus der undurchlässigen Deckschicht 9, dem Arzneimittelreservoir 10, der durchlässigen Polymerschicht 11 und der Hauthaftkleberschicht 12.

Fig. 5 zeigt einen Querschnitt durch ein rundes zweikammeriges Reservoirsystem gemäß Variante c ohne die abziehbare Schutzschicht. Es besteht aus der undurchlässigen Deckschicht 13, den beiden halbrunden Arzneimittelreservoiren 14 und 15, der durchlässigen Polymerschicht 16 und der Hauthaftkleberschicht 16.

Neben transdermalen therapeutischen Systemen eignen sich auch weitere galenische Zubereitungen zur transdermalen Applikation von 3-Keto-desogestrel.

Ein Emulsionsgel zur transdermalen Applikation besteht beispielsweise aus dem Wirkstoff oder Wirkstoffgemisch, penetrationsverstärkenden Mitteln, Emulgatoren (wobei ambiphile Vertreter der penetrationsverstärkenden Mittel als Emulgatoren dienen können) und gegebenenfalls Matrixbildnern. Eine typische Rezeptur besteht aus 0,1 - 25 Gewichtsprozent Wirkstoff oder Wirkstoffgemisch, 0-10 Gewichtsprozent Emulgator, 0-5 Gewichtsprozent Matrixbildner, 0 bis 50 Gewichtsprozent penetrationsverstärkenden Mitteln und Wasser zu 100 Gewichtsprozent. Das Mittel wird in üblicher Weise emulgiert, und erforderlichenfalls mit den üblichen Antioxidantien, Konservierungstoffen etc. versetzt.

Einphasige Gele erhält man beispielsweise durch Lösen oder Suspendieren des Wirkstoffs oder das Wirkstoffgemisches in Lösungsmitteln wie Wasser, niederen Alkoholen oder Mischungen derselben, gegebenenfalls unter Zusatz von penetrationsverstärkenden Mitteln und Verdicken mit Matrixbildnern.

Typische Rezepturen für solche Gele enthalten 0,01-25 Gewichtsprozent Wirkstoff oder Wirkstoffgemisch, 1-20 Gewichtsprozent Matrixbildner, 0 bis 40 Gewichtsprozent penetrationsverstärkende Mittel ergänzt mit dem Lösungsmittel zu 100 Gewichtsprozent.

Auch diese Gele können gewünschtenfalls Antioxidantien, Konservierungsstoffe etc. enthalten.

### Eine typische Sprayrezeptur ist beispielsweise folgende:

1-25 Gewichtsprozent Wirkstoff oder Wirkstoffgemisch, 0-20 Gewichtsprozent Matrixbildner, 0-60 Gewichtsprozent penetrationsverstärkende Mittel ergänzt mit Lösungsmitteln und gegebenenfalls Treibmitteln zu 100 %. Verwendet man Druckgaspackungen, so kann das Treibmittel entfallen.

Die erfindungsgemäßen 3-Keto-desogestrelhaltigen Mittel zur transdermalen Applikation können zur Behandlung der gleichen Erkrankungen angewendet werden, wie die vorbekannten, beispielsweise oral zu applizierenden Mittel die hochwirksame Gestagene enthalten. Darüberhinaus können die erfindungsgemäßen gegebenenfalls östrogenhaltigen Präparate auch zur Kontrazeption Anwendung finden. Besondere Vorteile haben die erfindungsgemäßen Mittel bei der Behandlung von Erkrankungen, die eine Langzeitbehandlung mit relativ hoher Dosierung der Wirkstoffe erfordern. Hier kann die Applikationsfrequenz wesentlich verringert werden und ein wesentlich gleichmäßiger Blutplasmaspielgel erzielt werden. Vorteilhaft ist ferner, daß gastrointestinale Nebenwirkungen nicht zu erwarten sind und bei östrogenhaltigen Kombinationspräparaten die erste Leberpassage umgangen wird und daß die Dosis an Östrogen vermindert werden kann.

Diese Vorteile lassen die östrogenfreie Monotherapeutika der vorliegenden Erfindung als besonders geeignet erscheinen um beispielsweise die Endometriose, gestagenabhängige Tumore, benigne Brusterkrankungen oder das prämerstruelle Syndrom zu behandeln.

Die transdermale Anwendung von Estrogenen in sequentieller oder kontinuierlicher Kombination mit 3-Keto-desogestrel bietet besondere Vorteile, beispielsweise zur Behandlung klimakterischer Beschwerden, zur Präventation der Osteoporose, zur Zyklusregulierung und zur Zyklusstabilisation.

Die nachfolgenden Ausführungsbeispiele dienen zur näheren Erläuterung der Erfindung. In ihnen wurden folgende Handelsprodukte verwendet:

Polyesterfolie von 0,074 mm Dicke (Skotchpak ® 1009 des Herstellers 3M;
Polypropylenfolie (Celgard ® 2500) des Herstellers Celanese, Linerfolie Skotchpak ® 1022 und 1360 vom Hersteller 3M; Transferkleber 9871 vom Hersteller 3M, Polyacrylester-Kleber vom Typ Sichello ® J 6610-21 des Herstellers Henkel KG, Silikonklebstoff vom Typ X-7-2960 des Herstellers Dow Corning und Hydroxypropylcellulose des Typs Klucel ® HXF des Herstellers Hercules, Polyisobutylen vom Typ Oppanol ® B 15 SF der Firma BASF AG.

### Beispiel 1

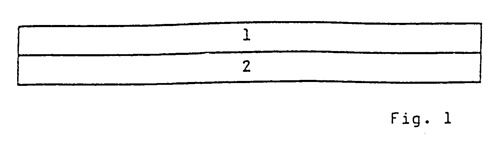

In 62,4 g einer 50 %igen Lösung von Silikonklebstoff in Benzin werden unter Rühren nacheinander
- 0,8 g: 3-Keto-desogestrel
- 8,0 g: 1,2 Propandiol
eingetragen. Nach Entgasen des Ansatzes wird die Mischung mittels einer Beschichtungsvorrichtung auf Polyesterfolie in der Weise aufgetragen, daß nach Entfernung des flüchtigen Lösemittels ein gleichmäßiger Film von 40 g/m² Feststoffauftrag entsteht. Anschließend wird mit einem fluorpolymerbeschichteten Polyester-Liner kaschiert. Das so erhaltene Laminat wird mittels einer Stanzvorrichtung in runde Einzelpflaster von 10 cm² Fläche geteilt und in Aluminiumfolie verpackt. Fig. 1 zeigt einen Querschnitt durch dieses Pflaster ohne Polyester-Liner. Das Pflaster haftet nach Abziehen der Liner-Folie auf der Haut.

Die Gehaltsbestimmung ergibt eine gleichmäßige Wirkstoftverteilung von 0,08 mg/cm² im Mittel.

### Beispiel 2

In 170 g einer 50 %igen Lösung von Polyacrylester-Klebstoff in Aceton/Benzin werden nacheinander
- 5,0 g: 3-Keto-desogestrel und
- 10,0 g: Isopropylmyristat
unter Rühren gelöst. Nach Entgasen des Ansatzes wird die Lösung mittels einer Beschichtungsvorrichtung auf Polyesterfolie in der Weise aufgetragen, daß nach Entfernung der flüchtigen Lösemittel ein gleichmäßiger Film von 100 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Fläche geteilt und in Aluminiumfolie verpackt. Das Pflaster haftet nach Abziehen der Liner-Folie auf der Haut.

Der Gehalt an Desogestrel beträgt im Mittel 0,5 mg/cm².

### Beispiel 3

Zu 112 g einer 50 %igen Lösung von Polyacrylester-Klebstoff in Aceton/Benzin werden nacheinander
- 3,5 g: Estradiol
- 3,5 g: 3-Keto-desogestrel und
- 7,0 g: 1,2-Propandiol mit 10 % 1-Dodecanol
unter Rühren gelöst bzw. suspendiert. Nach Entgasen des Ansatzes wird die Mischung mittels einer Beschichtungsvorrichtung auf Polyesterfolie in der Weise aufgetragen, daß nach Entfernung der flüchtigen Lösemittel ein gleichmäßiger Film von 70 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Fläche geteilt und in Aluminiumfolie verpackt. Das Pflaster haftet nach Abziehen der Liner-Folie auf der Haut.

Der Gehalt an Estradiol und 3-Keto-desogestrel liegt gleichermaßen bei je 0,35 mg/cm².

### Beispiel 4

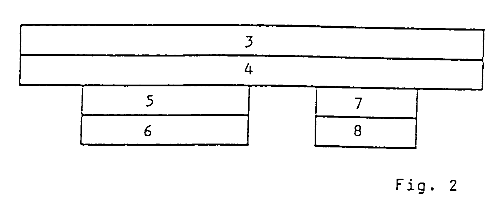

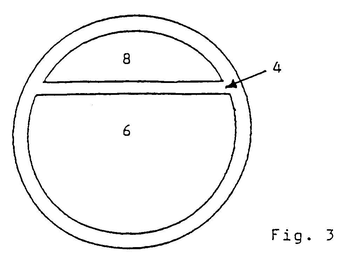

Analog Beispiel 1 werden zwei unterschiedliche segmentartige Matrixsysteme hergestellt, die die in Fig. 2 und 3 dargestellte Formgebung haben. Das Matrixsystem I besteht aus der mit einer Polyesterfolie 7 versehenen Matrixschicht 8 folgender Zusammensetzung
- 1,0 mg: 3-Keto-desogestrel
- 5,0 mg: Isopropylmyristat und
- 44 mg: Acrylatkleberfeststoff
und hat eine Fläche von 5 cm².

Das Matrixsystem II besteht aus der mit einer Polyesterfolie 5 versehenen Matrixschicht 6 folgender Zusammensetzung
- 2,0 mg: 17β-Estradiol
- 10,0 mg: Isopropylmyristat und
- 88 mg: Acrylatkleberfeststoffe
und hat eine Fläche von 10 cm².

Beide Matrixsysteme werden auf eine mit Hauthaftkleber beschichtete Leinwand aufgeklebt, wie Fig. 3 zeigt. Nach Kaschieren und Ausstanzen entstehen so Pflaster der in Fig. 2 und 3 gezeigten Art.

### Beispiel 5

Eine Polyesterfolie von 7,4 cm Durchmesser wird mittels Zug und Wärme so verformt, daß eine runde Ausbuchtung von 10 cm² Fläche entsteht. Diese wird mit 1 ml einer Suspension von
- 2,5 mg: Estradiol und
- 2,5 mg: 3-Keto-desogestrel
in 1,2-Propandiol, welches 10 % Laurinsäure enthält, gefüllt. Eine Polypropylen- oder Celluloseacetatbutyrat-Folie wird am Rand aufgeschweißt. Je nach Druck pro Zeiteinheit liegt die Siegeltemperatur zwischen 70 °C und 100 °C. Auf die durchlässige Polymerschicht wird Haftkleberfolie transferiert. Das Pflaster wird mit einem Liner versehen und in Aluminiumfolie verpackt.

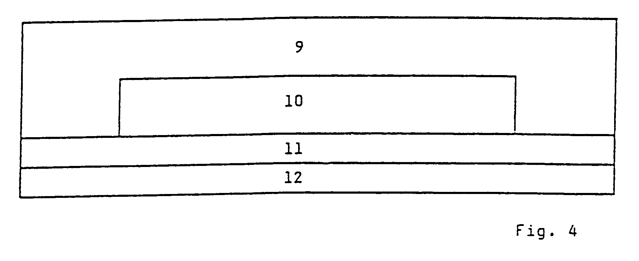

Fig. 4 zeigt einen Querschnitt durch ein Pflaster dieser Art ohne Liner.

Aus diesem Pflaster werden für beide Wirkstoffe gleichermaßen in-vitro-Freigaberaten in Wasser von 32 °C zwischen 0,02 bis 0,08 µg/cm²/Stunde erzielt.

### Beispiel 6

Analog Beispiel 5 wird eine Polyesterfolie so verformt, daß zwei halbrunde, durch einen Steg voneinander getrennte Ausbuchtungen von je 7,5 cm² Fläche entstehen.

Reservoir I wird mit 0,75 ml einer Suspension von
- 1,5 mg: 3-Keto-desogestrel
- in: 1,2-Propandiol
und Reservoir II mit 0,75 ml einer solchen von
- 3,0 mg: 17β-Estradiol
- in: 1,2-Propandiol
gefüllt. Die weitere Fertigstellung des Pflasters erfolgt wie in Beispiel 5 beschrieben.

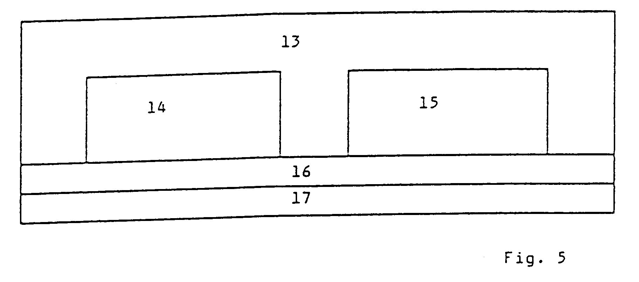

Fig. 5 zeigt einen Querschnitt durch ein derartiges Pflaster ohne Liner.

### Beispiel 7

In 76,78 g Ethanol (96 Vol %ig) oder Isopropanol werden nacheinander
- 0,2 g: 17β-Estradiol
- 0,02 g: 3-Keto-desogestrel
- 10,0 g: 1,2-Propandiol und
- 10,0 g: Isopropylmyristat
gelöst. Dann setzt man der Lösung 3 g Hydropropylcellulose zu und entfernt aus ihr die Luft. Nach 2 Stunden Quellzeit wird das Gel in Aluminiumtuben mit dreifacher Innenschutzlackierung abgefüllt.

Die Gehaltsbstimmung ergibt eine homogene Wirkstoffverteilung im Gel mit Werten von 95 % bis 105 % des Sollwertes.

### Beispiel 8

20,00 g 3-Keto-desogestrel werden in 1000 g Isopropylmyristat gelost, sterilfiltriert und unter aseptischen Bedingungen in Arzneiflaschen a 5 ml abgefüllt.

### Beispiel 9

In 112 g einer 50%igen Lösung von Polyisobutylen-Kunststoff (Oppanol ® B 15 SF der Firma BASF AG) in Aceton -Benzin werden nacheinander
- 3,5 g: Ethinylestradiol,
- 3,5 g: 3-Keto-desorgestrel und
- 7,0 g: Isopropylmyristat
unter Rühren eingearbeitet und aufbereitet wie in Beispiel 3 beschrieben.

## Patentansprüche

1. Mittel zur transdermalen Applikation, dadurch gekennzeichnet, daß es 3-Keto-desogestrel gegebenenfalls in Kombination mit einem oder mehreren Östrogen(en) enthält.

2. Mittel zur transdermalen Applikation gemäß Patentanspruch 1, dadurch gekennzeichnet, daß als Östrogen(e) Estradiol, Estriol, 17α-Ethinylestradiol, Mestranol, 14α, 17α-Ethanoestra-1,3,5(10)-trien-3β,17α-diol, 14α,17α-Ethanoestra-1,3,5(10)-trien-3β,16α,17α-triol oder Ester dieser Verbindungen verwendet werden.

3. Mittel zur transdermalen Applikation gemäß Patentanspruch 1 oder 2, dadurch gekennzeichnet, daß das Mittel ein transdermales therapeutisches System (TTS) ist.

4. Mittel zur transdermalen Applikation gemäß Patentanspruch 3, dadurch gekennzeichnet, daß das transdermale therapeutische System aus
a) einer undurchlässigen Deckschicht,
ein bis drei an der Deckschicht haftenden, das 3-Keto-desogestrel, gegebenenfalls das oder die Östrogen(e) und gewünschtenfalls penetrationsverstärkende Mittel enthaltende, für diese Komponenten durchlässigen selbsthaftenden oder von einem gewünschtenfalls penetrationsverstärkende Mittel enthaltenden Hauthaftkleber abgedecktem oder umgebenen Matrixschicht(en), einer abziehbaren Schutzschicht, oder
b) einer mit einem gewünschtenfalls penetrationsverstärkende Mittel enthaltenden Haftkleber versehenen Abdeckung,
ein bis drei (jeweils) eine Haftkleberumrandung unbedeckt lassende, mittels einer undurchlässigen Abdeckung an dem Haftkleber befestigten, das 3-Keto-desogestrel, gegebenenfalls das oder die Östrogen(e) und penetrationsverstärkende Mittel enthaltende Matrixschicht(en) und einer abziehbaren Schutzschicht, oder
c) einer undurchlässigen Deckschicht,
ein bis drei an oder in der Deckschicht befindlichen, das 3-Keto-desogestrel, gegebenenfalls das oder die Östrogen(e) und gewünschtenfalls penetrationsverstärkende Mittel enthaltenden Arzneimitelreservoir(e),
ein bis drei für diese Komponenten durchlässigen Polymerschicht(en) einer durchlässigen gegebenenfalls penetrationsverstärkende Mittel enthaltende Hauthaftkleber-Schicht und einer abziehbaren Schutzschicht besteht.

5. Mittel zur transdermalen Applikation gemäß Patentanspruch 4, dadurch gekennzeichnet, daß es eine wirkstoffhaltige Matrixschicht oder ein Arzneimittelreservoir enthält.

6. Mittel zur transdermalen Applikation gemäß Patentanspruch 4, dadurch gekennzeichnet, daß es zwei oder drei wirkstoffhaltige Matrixschichten oder Arzneimittelreservoire enthält.

7. Mittel zur transdermalen Applikation gemäß Patentanspruch 6, dadurch gekennzeichnet, daß die wirkstoffhaltigen Matrixschichten oder die Arzneimittelreservoire unterschiedliche Wirkstoffe enthalten.

8. Verwendung von 3-Keto-desogestrel gegebenerfalls in Kombination mit einem oder mehreren Östrogen(en) zur Herstellung eines Mittels für die transdermale Applikation des Wirkstoffes oder Wirkstoffgemisches.

9. Verwendung von 3-Keto-desogestrel in Kombination mit einem oder mehreren Östrogen(en) zur Herstellung eines Mittels gemäß Patentanspruch 8, dadurch gekennzeichnet, daß als Östrogen(e) Estradiol, Estriol, 17α-Ethinylestradiol,Mestranol, 14α, 17α-Ethanoestra-1,3,5(10)-trien-3β,17α-diol, 14α,17α-Ethanoestra-1,3,5(10)-trien-3β,16α,17α-triol oder Ester dieser Verbindungen verwendet werden.

10. Verwendung von 3-Keto-desogestrel gegebenenfalls in Kombination mit einem oder mehreren Östrogen(en) zur Herstellung eines Mittels gemäß Patentanspruch 8 oder 9, dadurch gekennzeichnet, daß das Mittel ein transdermales therapeutisches System (TTS) ist.

11. Verwendung von 3-Keto-desogestrel gegebenenfalls in Kombination mit einem oder mehreren Östrogen(en) zur Herstellung eines Mittels gemäß Patentanspruch 10, dadurch gekennzeichnet, daß es ein transdermales therapeutisches System gemäß Patentanspruch 4 bis 7 ist.

12. Verwendung von 3-Keto-desogestrel zur Herstellung eines östrogenfreien Mittels gemäß einem der Patentansprüche 1-7 zur Herstellung eines transdermalen therapeutischen Systems (TTS), zur Kontrazeption, zur Behandlung (a) der Endometriose, (b) von gestagenabhängigen Tumoren und (c) des prämenstruellen Syndroms sowie zur (d) Zyklusregulierung.

13. Verwendung von 3-Keto-desogestrel zur Herstellung eines Mittels gemäß einem der Patentansprüche 1 - 7 zur Herstellung eines transdermalen therapeutischen Systems (TTS) zur Kontrazeption, zur Behandlung (a) klimakterischer Beschwerden, (b) zur Prävention der Osteoporose, (c) zur Zyklusregulierung sowie (d) zur Zyklusstabilisation.

## Claims

1. Means for transdermal administration, characterised in that it contains 3-keto-desogestrel optionally in combination with one or more oestrogen(s).

2. Means for transdermal administration according to patent claim 1, characterised in that as oestrogen(s) there are used oestradiol, oestriol, 17α-ethynyloestradiol, mestranol, 14α,17α-ethano-oestra-1,3,5(10)-triene-3β,17α-diol, 14α,17α-ethano-oestra-1,3,5(10)-triene-3β,16α,17α-triol or esters of those compounds.

3. Means for transdermal administration according to patent claim 1 and 2, characterised in that the means is a transdermal therapeutic system (TTS).

4. Means for transdermal administration according to patent claim 3, characterised in that the transdermal therapeutic system consists of
a) an impermeable cover layer,
from one to three matrix layer(s) that adhere to the cover layer, that contain the 3-keto-desogestrel, optionally the oestrogen(s) and, if desired, penetration-enhancing agents and that are permeable to those components and are self-adhesive or are covered or surrounded by a skin adhesive which may, if desired, contain penetration-enhancing agents, and a removable protective layer, or
b) a covering provided with an adhesive which may, if desired, contain penetration-enhancing agents,
from one to three matrix layers(s) that (each) leave an adhesive border area uncovered, that are attached to the adhesive by means of an impermeable covering and that contain the 3-keto-desogestrel, optionally the oestrogen(s) and penetration-enhancing agents, and a removable protective layer, or
c) an impermeable cover layer,
from one to three medicament reservoir(s) that are located on or in the cover layer and that contain the 3-keto-desogestrel, optionally the oestrogen(s) and, if desired, penetration-enhancing agents,
from one to three polymer layer(s) permeable to those components,
a permeable skin adhesive layer that optionally contains penetration-enhancing agents, and a removable protective layer.

5. Means for transdermal administration according to patent claim 4, characterised in that it contains an active-ingredient-containing matrix layer or a medicament reservoir.

6. Means for transdermal administration according to patent claim 4, characterised in that it contains two or three active-ingredient-containing matrix layers or medicament reservoirs.

7. Means for transdermal administration according to patent claim 6, characterised in that the active-ingredient-containing matrix layers or the medicament reservoirs contain different active ingredients.

8. Use of 3-keto-desogestrel optionally in combination with one or more oestrogen(s) in the preparation of a means for the transdermal administration of the active ingredient or active ingredient mixture.

9. Use of 3-keto-desogestrel in combination with one or more oestrogen(s) in the preparation of a means according to patent claim 8, characterised in that there are used as oestrogen(s) oestradiol, oestriol, 17α-ethynyloestradiol, mestranol, 14α,17α-ethano-oestra-1,3,5(10)-triene-3β,17α-diol, 14α,17α-ethano-oestra-1,3,5(10)-triene-3β,16α,17α-trio or esters of those compounds.

10. Use of 3-keto-desogestrel optionally in combination with one or more oestrogen(s) in the preparation of a means according to patent claim 8 and 9, characterised in that the means is a transdermal therapeutic system (TTS).

11. Use of 3-keto-desogestrel optionally in combination with one or more oestrogen(s) in the preparation of a means according to patent claim 10, characterised in that it is a transdermal therapeutic system according to patent claim 4 to 7.

12. Use of 3-keto-desogestrel in the preparation of an oestrogen-free means according to patent claim 1 to 7 in the preparation of a transdermal therapeutic system (TTS), for the purpose of contraception, for the treatment (a) of endometriosis, (b) of gestagen-dependent tumours and (c) of pre-menstrual syndrome and (d) for the purpose of menstrual cycle regulation.

13. Use of 3-keto-desogestrel in the preparation of a means according to patent claim 1 to 7 in the preparation of a transdermal therapeutic system (TTS) for the purpose of contraception, for the treatment (a) of climacteric complaints, (b) in the prevention of osteoporosis, (c) for the purpose of menstrual cycle regulation and (d) for the purpose of menstrual cycle stabilisation.

## Revendications

1. Agent pour administration transdermique, caractérisé en ce qu'il contient du 3-céto-désogestrel, éventuellement en combinaison avec un ou plusieurs oestrogènes.

2. Agent pour administration transdermique selon la revendication 1, caractérisé en ce qu'on utilise en tant qu'oestrogènes l'oestradiol, le oestriol, le 17α-éthynyloestradiol, le mestranol, le 14α,17α-éthanoestra-1,3, 5(10)-triène-3β,17α-diol, le 14α,17α-éthanoestra-1,3, 5(10)-triène-3β,16α,17α-triol, ou des esters de ces composés.

3. Agent pour administration transdermique selon la revendication 1 ou 2, caractérisé en ce que l'agent est un système thérapeutique transdermique (TTS).

4. Agent pour administration transdermique selon la revendication 3, caractérisé en ce que le système thérapeutique transdermique est constitué :
a) d'une couche de couverture imperméable,
d'une à trois couches de matrice adhérant à la couche de couverture, contenant du 3-céto-désogestrel, éventuellement le ou les oestrogènes et si possible des agents renforçant la pénétration, perméables à ces constituants, auto-adhérentes, ou encore recouvertes ou entourées par un adhésif cutané de contact contenant si possible des agents renforçant la pénétration ;
d'une couche de couverture pelable, ou bien
b) d'un recouvrement pourvu d'un adhésif de contact contenant si possible des agents renforçant la pénétration,
d'une à trois couches de matrice, laissant chacune non recouvert un bord de l'adhésif de contact, fixées à l'adhésif à l'aide d'un recouvrement, et contenant le 3-céto-désogestrel, éventuellement le ou les oestrogènes et les agents renforçant la pénétration,
et d'une couche de protection pelable, ou bien
c) d'une couche de couverture imperméable,
d'un à trois réservoirs de médicament, se trouvant contre ou dans la couche de couverture, contenant le 3-céto-désogestrel, éventuellement le ou les oestrogènes et si possible les agents renforçant la pénétration,
d'une à trois couches de polymère perméables à ces constituants,
d'une couche d'un adhésif de contact pour la peau, perméable et contenant éventuellement des agents renforçant la pénétration,
et d'une couche de protection pelable.

5. Agent pour administration transdermique selon la revendication 4, caractérisé en ce qu'il contient une couche de matrice contenant des principes actifs, ou un réservoir à médicaments.

6. Agent pour administration transdermique selon la revendication 4, caractérisé en ce qu'il contient deux ou trois couches de matrice contenant des principes actifs, ou réservoirs à médicaments.

7. Agent pour administration transdermique selon la revendication 6, caractérisé en ce que les couches de matrice contenant des principes actifs ou les réservoirs à médicaments contiennent différents principes actifs.

8. Utilisation de 3-céto-désogestrel, éventuellement en combinaison avec un ou plusieurs oestrogènes, pour préparer un agent pour administration transdermique du principe actif ou du mélange de principes actifs.

9. Utilisation de 3-céto-désogestrel en combinaison avec un ou plusieurs oestrogènes pour préparer un agent selon la revendication 8, caractérisé en ce qu'on utilise en tant qu'oestrogènes l'oestradiol, le oestriol, le 17α-éthynyloestradiol, le mestranol, le 14α,17α-éthanoestra-1,3,5(10)-triène-3β,17α-diol, le 14α,17α-éthanoestra-1,3,5(10)-triène-3β,16α,17α-triol, ou des esters de ces composés.

10. Utilisation de 3-céto-désogestrel, éventuellement en combinaison avec un ou plusieurs oestrogènes, pour préparer un agent selon les revendications 8 et 9, caractérisé en ce que l'agent est un système thérapeutique transdermique (TTS).

11. Utilisation de 3-céto-désogestrel, éventuellement en combinaison avec un ou plusieurs oestrogènes, pour préparer un agent selon la revendication 10, caractérisé en ce qu'il s'agit d'un système thérapeutique transdermique selon les revendications 4 à 7.

12. Utilisation de 3-céto-désogestrel pour préparer un agent sans oestrogènes selon les revendications 1 à 7, pour préparer un système thérapeutique transdermique (TTS), pour la contraception, pour le traitement (a) de l'endométriose, (b) des tumeurs dépendant des gestagènes, et (c) du syndrome prémenstruels, et (d) pour la régulation des cycles.

13. Utilisation de 3-céto-désogestrel pour préparer un agent selon les revendications 1 à 7 pour préparer un système thérapeutique transdermique (TTS), pour la contraception, pour le traitement (a) des troubles de la ménopause, (b) pour la prévention de l'ostéoporose, (c) pour la régulation des cycles, et (d) pour la stabilisation des cycles.
